Europäisches Patentamt

(19) European Patent Office

Office européen des brevets

(11) Publication number: **0 204 525**
**B1**

(12) # EUROPEAN PATENT SPECIFICATION

(45) Date of publication of the patent specification:
06.09.89

(51) Int. Cl.⁴: **A 61 N 1/32**

(21) Application number: **86304129.9**

(22) Date of filing: **30.05.86**

(54) Low-frequency therapeutic device.

(30) Priority: **30.05.85 JP 115368/85**

(43) Date of publication of application:
**10.12.86 Bulletin 86/50**

(45) Publication of the grant of the patent:
**06.09.89 Bulletin 89/36**

(84) Designated Contracting States:
**DE FR GB IT**

(56) References cited:
**AU-B- 434 062**
**DD-A- 24 660**
**DE-A- 2 058 950**
**DE-A- 2 356 481**
**DE-A- 3 212 706**
**FR-A- 2 137 352**
**GB-A- 1 333 238**
**US-A- 3 585 991**
**US-A- 3 822 693**

(73) Proprietor: **C. S Kogyo Company, Ltd., 9-5, Nihonbashi Kayabacho 2-chome, Chuo-ku Tokyo (JP)**

(72) Inventor: **Kishimoto, Keisuke, C. S Kogyo Co. Ltd. 9-5, Nihonbashi Kayabacho 2-ch, Chuo-ku Tokyo (JP)**

(74) Representative: **Tomlinson, Kerry John et al, Frank B. Dehn & Co. European Patent Attorneys Imperial House 15-19 Kingsway, London WC2B 6UZ (GB)**

## Description

The present invention relates to a low-frequency therapeutic device for applying low-frequency electric energy to a local area in a human body through the skin to treat the local area for recovery from fatigue, improved blood circulation, aesthetic massage, shaping-up, or other therapeutic purposes.

It has widely been practised to treat diseased areas in human bodies by applying low-frequency energy having a frequency ranging from 1 to 500 Hz. It is known that the frequencies used in such low-frequency therapy have different therapeutic effects. For example, relatively low frequencies up to 70 Hz are effective in twitching muscles and hence have high kinetic effects. Relatively high frequencies higher than 100 Hz have high stimulative effects for stimulating superficial fascias and muscles. Therefore, different low frequencies should be employed for treating different diseases.

One conventional low-frequency therapeutic device has an oscillator for producing low frequencies ranging from 1 Hz to 500 Hz in an continuously or incrementally variable manner. In treating a diseased area, a low frequency is selected or low frequencies are varied at fixed time intervals in view of the type of the disease, the age of the patient, the condition of the muscles, the amount of fat in the diseased area, the body configuration of the patient, the susceptiblility of the patient, and other factors. DD-A-24 660 discloses stimulation apparatus comprising two pulse generators whose outputs are fed in parallel to a muscle.

There is no definite correlation between the low frequencies and the disease types. The frequencies are selected at most to distinguish between the kinetic and stimulative effects. For many purposes such as recovery from fatigue, improved blood circulation, aesthetic massage, and shaping-up, it is sometimes preferable to give the diseased area both kinetic and stimulative energies for greater therapeutic effects.

However, the conventional low-frequency therapeutic device cannot supply therapeutic currents of different frequencies from the same path held in contact with the diseased area. It has therefore been customary in actual treatments using the low-frequency therapeutic device for skilled specialists to make appropriate treatment plans in view of the type of the disease, the age of the patient, the condition of the muscles, the amount of fat in the diseased area, the body configuration of the patient, the susceptibility of the patient, and other factors.

## Summary of the invention

It is an object of the present invention to provide a low-frequency therapeutic device capable of supplying a diseased area with a plurality of stimulative currents of different frequencies simultaneously from the same pad for subjecting the diseased area to a plurality of different treatments at the same time for increased therapeutic effects.

Another object of the present invention is to provide a low-frequency therapeutic device capable of supplying a diseased area with a plurality of stimulative currents of different frequencies simultaneously from the same pad, of allowing the patient to listen to music for stimulating the patient with the varying sound intensities and rhythm of the music to keep a patient treated for a long period of time from getting bored, and of increasing therapeutic effects due to the psychological effect of the music.

According to the present invention, there is provided a low-frequency therapeutic device which essentially includes a plurality of oscillator circuits for generating output pulses of different frequencies, a pulse mixing circuit for mixing the output pulses from the oscillator circuits to produce mixed output pulses, and at least one pad for supplying a mixed stimulative current composed of stimulative currents of different frequencies in response to the mixed output pulses. The low-frequency therapeutic device can simultaneously treat a diseased area with a plurality of therapeutic effects. The low-frequency therapeutic device may additionally include an acoustic device for producing a music signal dependent on the waveform of sounds of music, a reproducer for generating the sounds of music in response to the music signal, and a signal output circuit responsive to the music signal having an amplitude greater than a certain level for generating an output signal dependent on the music signal, the oscillator circuits being responsive to the output signal for generating the output pulses of different frequencies. During treatment, the patient can listen to the music, and the mixed stimulative current supplied from the pad is dependent on the varying sound intensities and rhythm of the music.

The oscillator circuits employed in the low-frequency therapeutic device may be of any conventional design capable of producing output pulses of low frequencies. The oscillator circuits of the invention may be of the same arrangement or different arrangements.

As many oscillator circuits as there are frequencies to be used in the low-frequency therapeutic device may be employed for producing output pulses of prescribed frequencies. Where two oscillator cicuits, for example, are employed to supply two stimulative currents simultaneously from the pad, one of the oscillator circuits may produce output pulses of frequencies in the range of from about 5 to 60 Hz in a continuously or incrementally variable manner, and the other oscillator circuit may produce output pulses of frequencies higher than about 100 Hz in a continuously or incrementally variable manner, so that the mixed stimulative current is composed of stimulative currents of desired frequencies.

The pulse mixing circuit for mixing the output pulses from the oscillator circuits into the mixed output pulses may also be of a conventional arrangement. For example, the pulse mixing circuit may comprise a circuit used in an acoustic reproducer for mixing signals from a plurality of microphones, or a circuit for mixing signals from a microphone and an acoustic reproducer.

After the output pulses from the oscillator circuits have been mixed by the pulse mixing circuit, it is impossible to adjust the output pulses before they are mixed together.

In the actual low-frequency therapeutic device, the output pulses from the oscillator circuits are applied to the pad through a pulse-width adjusting circuit, a differentiator, an output amplifier, an output transformer, and an output voltage regulator. The pulse mixing circuit may be located anywhere between the oscillator circuits and the pad, and the position thereof may be determined in actually designing the low-frequency therapeutic device.

The pad that is responsive to the mixed output pulses from the pulse mixing circuit for supplying the mixed stimulative current may be of any construction capable of applying the mixed output pulses to a diseased area of the patient for passing the stimulative current therethrough. For example, the pad may comprise an anode for passing the stimulative current to the diseased area and a cathode for receiving the stimulative current from the diseased area.

To add the varying sound intensities and rhythm of the music to the mixed stimulative current, the oscillator circuits are controlled by the output signal from the signal output circuit supplied with the music signal from the acoustic device.

The acoustic device employed may be a tape deck, a record player, a cassette tape deck, a radio receiver, or the like. Where the music signal from the acoustic device is small in level, it may be amplified by a low-frequency amplifier and the amplified signal bay be applied to the reproducer and the signal output circuit.

The reproducer may comprise a conventional transducer such as an earphone or a loudspeaker.

The signal output circuit may be a circuit capable of issuing singals dependent on music sounds in response to the amplitudes and/or frequencies of the music sounds. For example, the signal output circuit may comprise a Schmitt trigger circuit energizable only in response to a music signal of an amplitude higher than a certain level for producing output pulses, or a timing signal generator for producing output pulses by comparing a music signal obtained by integrating a music signal of an amplitude higher than a certain level with a reference level signal.

The above and other objects, features and advantages of the present invention will become more apparent from the following description when taken in conjunction with the accompanying drawings in which preferred embodiments of the present invention are shown by way of illustrative example.

Brief description of the drawings

Fig. 1 is a block diagram of a low-frequency therapeutic device according to a first embodiment of the present invention;

Fig. 2 is a diagram showing the waveform of mixed pulses issued from a pulse mixing circuit in the low-frequency therapeutic device shown in Fig. 1;

Fig. 3 is a block diagram of a low-frequency therapeutic device according to a second embodiment of the present invention; and

Fig. 4 is a block diagram of a signal output circuit in the low-frequency therapeutic device shown in Fig. 3.

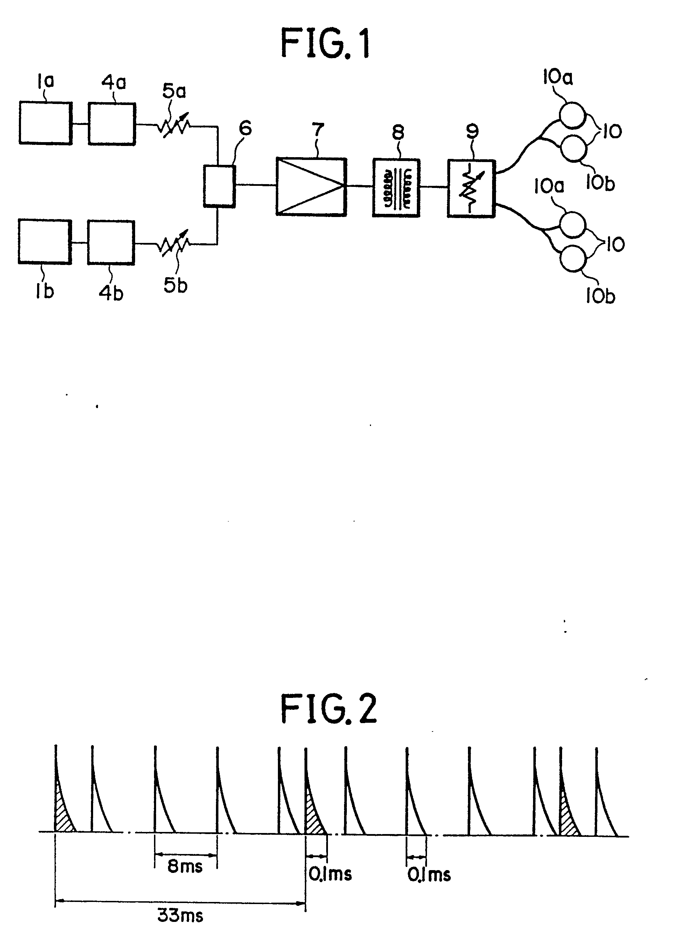

Fig. 1 shows a low-frequency therapeutic device according to a first embodiment of the present invention.

The low-frequency therapeutic device includes a pair of oscillator circuits 1a, 1b. Each oscillator circuit 1a, 1b in this embodiment generates output pulses of different frequencies which are set to prescribed values previously, and intermittently generates output pulses under the control of an output signal from a generating circuit of an intermittent signal (not shown).

The output pulses generated by the oscillator circuits 1a, 1b are differentiated and adjusted in their pulse widths by a pair of differentiators 4a, 4b respectively. The output pulses of the differentiated waveforms with their pulse widths adjusted are applied respectively to a pair of output voltage regulators 5a, 5b which regulate the output voltages of the output pulses. The output pulses the voltages of which are regulated to desired values are then applied to a pulse mixing circuit 6 which produces mixed output pulses.

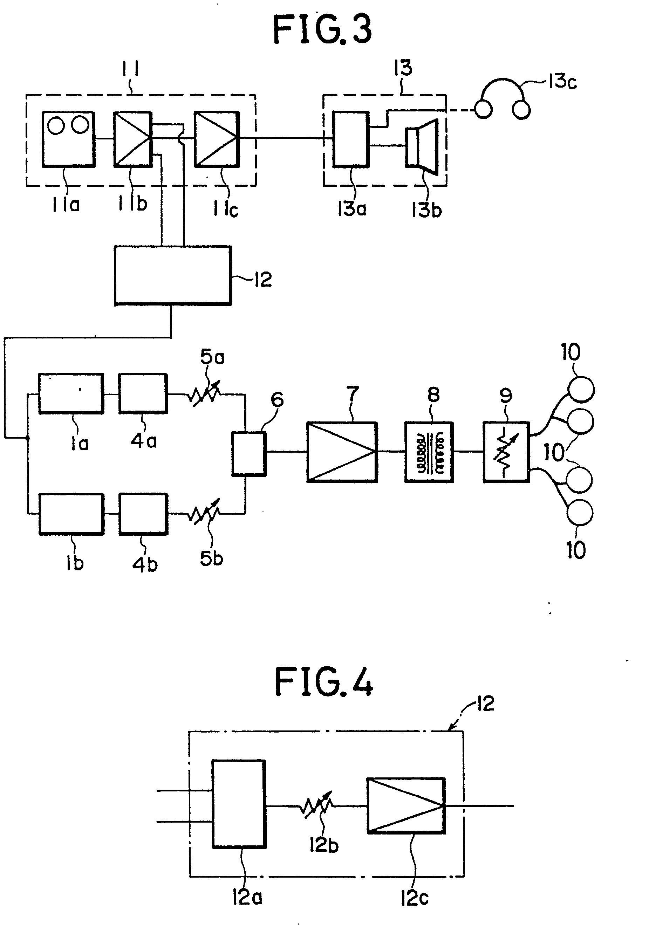

The mixed output pulses may have a waveform such as shown in Fig. 2. In the example of Fig. 3, the oscillator circuit 1a produces output pulses of a frequency of 30 Hz and the oscillator circuit 1b produces output pulses of a frequency of 120 Hz. These output pulses are processed by the differentiators 4a, 4b to have pulse widths of 0,1 ms, and regulated by the output voltage regulators 5a, 5b to have the same output voltages. As shown in Fig. 2, the output pulses originating from the oscillator circuit 1a occur at intervals of 33 ms, and the output pulses originating from the oscillator circuit 1b occur at intervals of 8 ms. The frequencies, pulse widths, and output voltages of the output pulses from the oscillator circuits 1a, 1b may appropriately be selected in view of the type of the disease to be treated, the position of the diseased area, and other elements. Only the pulse widths and output voltages may be adjustable while the frequencies may be fixed, or only the pulse widths or the output voltages may be adjusted or set to prescribed values.

The mixed output pulses from the pulse mixing circuit 6 are delivered through an output amplifier 7, an output transformer 8, and an output voltage regulator 9 to the anodes 10a and cathodes 10b of pads 10, from which a mixed stimulative current composed of two stimulative currents based on the output pulses from the oscillator circuits 1a, 1b are supplied to the diseased area of the patient.

According to the low-frequency therapeutic device of the first embodiment, the plurality of stimulative currents can simultaneously be supplied from one pad by mixing the output pulses of different frequencies. Therefore, the muscle twitching effect or kinetic effect due to the stimulative current based on the output pulses of a relatively low frequency, and the stimulative effect due to the stimulative current based on the output pulses of a relatively high frequency for stimulating superficial fascias and muscles can simultaneously be applied from the same pad to the diseased area. The diseased area can therefore be massaged and beaten at the same time, or pushed and massaged or beaten at the same time for improved therapeutic effects.

Figs. 3 and 4 show a low-frequency therapeutic device according to a second embodiment for adding varying sound intensities and rhythm of music to a mixed stimulative current to be supplied to a diseased area.

As illustrated in Fig. 3, the oscillator circuits 1a, 1b are controlled by an acoustic device 11 for issuing a music signal having a music sound waveform and a signal output circuit 12 for issuing an output signal responsive to the music signal having an amplitude greater than a certain level. A reproducer 13 for producing music sounds in response to the music signal is connected to the acoustic device 11.

The acoustic device 11 is composed of a stereophonic tape deck 11a, a preamplifier 11b, and an output amplifier 11c. The reproducer 13 comprises a headphone protector 13a, a loudspeaker 13b, and a headphone 13c. As shown in Fig. 4, the signal output circuit 12 has a signal mixing circuit 12a for mixing two music signals from the stereophonic tape deck 11a, a level adjuster 12b capable of setting a certain level at which the circuit responds to the music signal having an amplitude greater than a certain level for generating an output signal dependent on the music signal, and an amplifier 12c which amplifies the output so as to make it exactly the right level to operate each oscillator circuit 1a, 1b.

By thus controlling the oscillator circuits 1a, 1b with the music signals from the acoustic device 11, the output signals produced by the oscillator circuits 1a, 1b are composed of output pulses dependent on the varying sound intensities and rhythm of the music. Therefore, the diseased area being treated of the patient can be supplied with a mixed stimulative current which varies with the varying sound intensities and rhythm of the music. The patient can thus be given stimulation commensurate with the music while he or she is listening to the music.

With the low-frequency therapeutic device according to the second embodiment, the diseased area is supplied with a mixed stimulative current composed of a plurality of stimulative currents of different frequencies from the same pad by mixing a plurality of output pulses of different frequencies for attaining improved therapeutic effects. At the same time, the patient being treated can listen to music while he or she is subjected to stimulation dependent on the varying sound intensities and rhythm of the music. Therefore, the patient can be treated continously for a long period of time without getting bored, and the therapeutic effects can be increased due to the psychological effect of the music.

Although certain preferred embodiments have been shown and described, it should be understood that many changes and modifications may be made therein without departing from the scope of the appended claims.

## Claims

1. A low-frequency therapeutic device comprising a plurality of oscillator circuits (1a, 1b) for generating output pulses of different frequencies, a pulse mixing circuit (6) for mixing the output pulses from said oscillator circuits to produce mixed output pulses, and at least one pad (10) for supplying a mixed stimulative current composed of stimulative currents of different frequencies in response to said mixed output pulses.

2. A low-frequency therapeutic device according to claim 1, wherein each of said oscillator circuits has an output voltage regulator (5a, 5b) for regulating the output voltage of the produced output pulses.

3. A low-frequency therapeutic device comprising an acoustic (11) device for producing a music signal dependent on the waveform of sounds of music, a reproducer (13) for generating the sounds of music in response to said music signal, a signal output circuit (12) responsive to the music signal having an amplitude greater than a certain level for generating an output signal dependent on said music signal, a plurality of oscillator circuits (1a, 1b) for generating output pulses of different frequencies in response to said output signal, a pulse mixing circuit (6) for mixing the output pulses from said oscillator circuits to produce mixed output pulses, and at least one pad (10) for supplying a mixed stimulative current composed of stimulative currents of different frequencies in response to said mixed output pulses.

4. A low-frequency therapeutic device according to claim 3, wherein each of said oscillator circuits has an output voltage regulator (5a, 5b) for regulating the output voltage of the produced output pulses.

## Patentansprüche

1. Niederfrequenz-Therapiegerät mit mehreren Oszillatorschaltungen (1a, 1b) zum Erzeugen von Ausgangsimpulsen unterschiedlicher Frequenzen, einer Impuls-Mischschaltung (6) zum Mischen der Ausgangsimpulse von den Oszillatorschaltungen, um gemischte Ausgangsimpulse zu erzeugen, und zumindest einer Anschlussfläche (10) zum Zuführen eines gemischten stimulierenden Stroms, der aus stimulierenden Strömen unterschiedlicher Frequenzen zusammengesetzt ist, in Antwort auf die gemischten Ausgangsimpulse.

2. Niederfrequenz-Therapiegerät nach Anspruch 1, wobei jede der Oszillatorschaltungen einen Ausgangsspannungsregler (5a, 5b) zum Einstellen der Ausgangsspannung der erzeugten Ausgangsimpulse hat.

3. Niederfrequenz-Therapiegerät mit einer akustischen Vorrichtung (11) zum Erzeugen eines Musiksignals in Abhängigkeit von der Wellenform des Klangs der Musik, einem Wiedergabegerät (13) zum Erzeugen der Musikklänge in Antwort auf das Musiksignal, einer Signalausgangsschaltung (12), die auf das Musiksignal anspricht, das eine Amplitude grösser als ein bestimmter Pegel hat, um ein Ausgangssignal in Abhängigkeit von dem Musiksignal zu erzeugen, mehreren Oszillatorschaltungen (1a, 1b) zum Erzeugen von Ausgangsimpulsen unterschiedlicher Frequenzen in Antwort auf das Ausgangssignal, einer Impuls-Mischschaltung (6) zum Mischen der Ausgangsimpulse von den Oszillatorschaltungen, um gemischte Ausgangsimpulse zu erzeugen, und zumindest einer Anschlussfläche (10) zum Zuführen eines gemischten stimulierenden Stromes, der aus stimulierenden Strömen unterschiedlicher Frequenzen zusammengesetzt ist, in Antwort auf die gemischten Ausgangsimpulse.

4. Niederfrequenz-Therapiegerät nach Anspruch 3, wobei jede der Oszillatorschaltungen einen Ausgangsspannungsregler (5a, 5b) zum Einstellen der Ausgangsspannung der erzeugten Ausgangsimpulse hat.

**Revendications**

1. Dispositif thérapeutique à basses fréquences comprenant une pluralité de circuits oscillateurs (1a, 1b) pour générer des impulsions de sortie de fréquence différentes, un circuit mélangeur d'impulsions (6) pour le mélange d'impulsions de sortie provenant desdits circuits oscillateurs afin de produire des impulsions de sortie mélangées, et au moins une pastille (10) pour fournir un courant stimulant mélangé composé de courants stimulants de fréquences différentes en réponse auxdites impulsions de sortie mélangées.

2. Dispositif thérapeutique à basses fréquences selon la revendication 1, dans lequel chacun desdits circuits oscillateurs comporte un circuit de réglage de tension de sortie (5a, 5b) servant à régler la tension de sortie des impulsions de sortie produites.

3. Dispositif thérapeutique à basse fréquences comprenant un dispositif acoustique (11) pour produire un signal musical fonction de la forme d'onde de sons musicaux, un reproducteur de son (13) servant à générer les sons musicaux en réponse audit signal musical, un circuit de sortie de signal (12) sensible au signal musical ayant une amplitude supérieure à un certain niveau servant à générer un signal de sortie fonction dudit signal musical, une pluralité de circuits oscillateurs (1a, 1b) servant à générer des impulsions de sortie de fréquences différentes en réponse audit signal de sortie, un circuit mélangeur d'impulsions (6) servant à mélanger les impulsions de sortie provenant desdits circuits oscillateurs pour produire des impulsions de sortie mélangées, et au moins une pastille (10) pour fournir un courant stimulant mélangé composé de courants stimulants de fréquences différentes en réponse auxdites impulsions de sortie mélangées.

4. Dispositif thérapeutique à basses fréquences selon la revendication 3, dans lequel chacun desdits circuits oscillateurs comporte un circuit de réglage de tension de sortie (5a, 5b) pour régler la tension de sortie des impulsions de sortie produites.

1/2

# FIG.1

# FIG.2

8ms

0.1ms

0.1ms

33ms

2/2

# FIG.3

# FIG.4